# EUROPEAN PATENT APPLICATION

(11) **EP 3 150 648 A1**
(43) Date of publication of application: **05.04.2017**
(21) Application number: 15187819.6
(22) Date of filing: 01.10.2015
(51) Int. Cl.: C08G 18/48, C08G 18/75, C08G 18/08, C08G 18/28, C09D 175/16, C08G 18/38, C09J 175/16, C08L 75/16, A61K 8/00, C08G 18/66, C08G 18/12, C11D 1/00

(54) **AQUEOUS FUNCTIONALIZED POLYURETHANE DISPERSIONS**

(71) Applicant: Henkel AG & Co. KGaA, 40589 Düsseldorf (DE); Max-Planck-Gesellschaft zur Förderung der Wissenschaften e.V., 80539 München (DE)
(72) Inventor: TADEN, Andreas, 40597 Düsseldorf (DE); KELLER, Hannes, 40227 Düsseldorf (DE); LANDFESTER, Katharina, 55122 Mainz (DE)

(57) **Abstract**

The present invention relates to a process for manufacturing an aqueous polyurethane dispersion (PUD) comprising at least one diene-functionalized polyurethane and at least one dienophile-functionalized polyurethane, the process comprising (1)(a) providing a first NCO-functional polyurethane prepolymer and reacting said first NCO-functional polyurethane prepolymer with a compound comprising at least one NCO-reactive group and at least one diene group to obtain a diene-functionalized polyurethane; or (1)(b) reacting a first polyisocyanate with a compound comprising at least two NCO-reactive groups and at least one diene group to obtain a diene-functionalized polyurethane; (2) dispersing said diene-functionalized polyurethane into a continuous aqueous phase to form a diene-functionalized polyurethane dispersion; (3)(a) providing a second NCO-functional polyurethane prepolymer and reacting said second NCO-functional polyurethane prepolymer with a compound comprising at least one NCO-reactive group and at least one dienophile group to obtain a dienophile-functionalized polyurethane; or (3)(b) reacting a second polyisocyanate with a compound comprising at least two NCO-reactive groups and at least one dienophile group to obtain a dienophile-functionalized polyurethane; (4) dispersing said dienophile-functionalized polyurethane into a continuous aqueous phase to form a dienophile-functionalized polyurethane dispersion; and (5) combining the diene-functionalized polyurethane dispersion and the dienophile-functionalized polyurethane dispersion, thereby forming the aqueous polyurethane polymer dispersion. Also encompassed are the thus produced dispersions, compositions containing them and their use as coatings, adhesives, laundry detergents, textile treatment agents and cosmetics.

## Description

The present invention relates to aqueous functionalized polyurethane dispersions that can be used as adhesives and coatings. They provide for a versatile system that forms cross-links by a Diels-Alder cycloaddition reaction between separate polymer chains upon drying. As the reaction is reversible at elevated temperature the resulting materials are thermoresponsive and dissociate into the separate polymer chains at elevated temperatures. The materials are thus suited for the production of self-healing polymer films and for bond/debond-on-command adhesives. Also encompassed are processes for their production, compositions containing them and their use as coatings and adhesives.

One versatile class of polymers which is often used in adhesives and coatings consists of polyurethanes (PUs). PUs are variable in structure, they may incorporate crystalline domains, charged moieties or water-swellable segments, conferring properties like heat-activation, charge interactions and dispersibility in water. Of particular interest are waterborne polyurethane dispersions (PUDs). PUDs can be synthesized from a wide variety of polyols by reacting them with polyisocyanates to yield water-dispersible polymers with very interesting properties in the films formed thereof, such as partial crystallinity, elasticity, and high modulus. The first step in the synthesis most often consists of a polyaddition reaction between the polyols and the polyisocyanate monomers to form a PU prepolymer. Commonly, polyols with charged groups and with water-swellable moieties are used in order to generate so-called "self-stabilizing" structures, i.e. which can be dispersed in water without the addition of a surfactant. Through addition of a slight excess of isocyanate with regard to alcohol functionalities, a prepolymer of moderate molecular weight with NCO end groups is obtained. These end groups are typically used for a chain extension after dispersion to increase the molecular weight of the PU chains.

For most applications, PUDs are dried and used in form of polymer films. During film formation, water evaporates until the polymer particles come into contact with each other. Upon further evaporation of water the spherical particles undergo deformation with the last step being interdiffusion of the polymer chains. Such interdiffusion requires sufficient mobility of the polymer chains and therefore is typically limited to linear or branched polymers, excluding polymer networks. To achieve sufficient mechanical stability, in particular in the inter-particle space, it is common to post-cure the polymer film by cross-linkers that strengthen the inter-particle bonding. For this post-curing it is known to add hydrophilically modified polyisocyanates to the dispersions, which react with remaining hydroxyl and amine groups of the polyurethanes. Due to the potential hydrolysis of the isocyanates, they can only be added directly before use of the dispersion. Another known approach is the use of a hardener dispersion comprising hydrophobic polyisocyanates and hydrophilic polyols which is mixed with the PUD directly before use. It is further known to use latent reactive systems, which employ microparticles containing isocyanates. These have the drawback that it is difficult to ensure a homogeneous distribution in the dispersion. For coatings, sometimes blocked isocyanates are used. These suffer from the drawback that high deblocking temperatures of 120 to 200°C are necessary for their activation.

Although there are numerous systems known to cross-link PU films, there is still need for improved systems that overcome some or all of the drawbacks of existing systems. The present invention addresses this need by providing functionalized PUDs that are latent reactive in that they are stable under the typical storage conditions but after application and drying of the polymer films the cross-linking reaction automatically occurs.

The present invention is based on the inventors' finding that diene-functionalized PU particles can be mixed with dienophile-functionalized PU particles to form a stable aqueous dispersion of the particles, with a cross-linking reaction (Diels-Alder cycloaddition reaction) between the diene and dienophile only occurring upon drying of the dispersion when the particles come into close contact with each other. The respective dispersions are thus latent reactive without exhibiting the stability problems of other known systems.

In a first aspect, the present invention thus relates to a process for manufacturing an aqueous polyurethane polymer dispersion (PUD) comprising at least one diene-functionalized polyurethane and at least one dienophile-functionalized polyurethane, the process comprising
(1)
   (a) providing a first NCO-functional polyurethane prepolymer and reacting said first NCO-functional polyurethane prepolymer with a compound comprising at least one NCO-reactive group and at least one diene group, preferably a compound comprising one hydroxyl and at least one maleimide group, to obtain a diene-functionalized polyurethane; or
   (b) reacting a first polyisocyanate with a compound comprising at least two NCO-reactive groups and at least one diene group, preferably a compound comprising at least two hydroxyl and at least one diene group, preferably maleimide group, to obtain a diene-functionalized polyurethane;
(2) dispersing said diene-functionalized polyurethane into a continuous aqueous phase to form a diene-functionalized polyurethane dispersion;
(3)
   (a) providing a second NCO-functional polyurethane prepolymer and reacting said second NCO-functional polyurethane prepolymer with a compound comprising at least one NCO-reactive group and at least one dienophile group, preferably a compound comprising one hydroxyl and at least one furan group, to obtain a dienophile-functionalized polyurethane; or
   (b) reacting a second polyisocyanate with a compound comprising at least two NCO-reactive groups and at least one dienophile group, preferably a compound comprising at least two hydroxyl and at least one dienophile group, preferably furan group, to obtain a dienophile-functionalized polyurethane;
(4) dispersing said dienophile-functionalized polyurethane into a continuous aqueous phase to form a dienophile-functionalized polyurethane dispersion; and
(5) combining the diene-functionalized polyurethane dispersion and the dienophile-functionalized polyurethane dispersion, thereby forming the aqueous polyurethane polymer dispersion.

In another aspect, the invention relates to the aqueous functionalized polyurethane polymer dispersion obtainable according to the process described herein.

Further aspects of the invention relate to compositions, in particular adhesive compositions, which contain the aqueous functionalized polyurethane polymer dispersion disclosed herein and the use of the aqueous functionalized polyurethane polymer dispersion in adhesive and coating compositions.

"One or more", as used herein, relates to at least one and comprises 1, 2, 3, 4, 5, 6, 7, 8, 9 or more of the referenced species. Similarly, "at least one" means one or more, i.e. 1, 2, 3, 4, 5, 6, 7, 8, 9 or more. "At least one", as used herein in relation to any component, refers to the number of chemically different molecules, i.e. to the number of different types of the referenced species, but not to the total number of molecules. For example, "at least one polyol" means that at least one type of molecule falling within the definition for a polyol is used but that also two or more different molecule types falling within this definition can be present, but does not mean that only one molecule of said polyol is present.

If reference is made herein to a molecular weight of a PU polymer or its components, this reference refers to the number average molecular weight Mₙ, if not explicitly stated otherwise. The number average molecular weight Mₙ can be calculated based on end group analysis (OH numbers according to DIN 53240) or can be determined by gel permeation chromatography (GPC) according to DIN 55672-1:2007-08 with THF as the eluent. If not stated otherwise, all given molecular weights are those determined by end group analysis. The weight average molecular weight M_{w} can be determined by GPC, as described for Mₙ.

All percentages given herein in relation to the compositions or formulations relate to weight % relative to the total weight of the respective composition or formula, if not explicitly stated otherwise.

"NCO-functional", as used herein, relates to a prepolymer that has free isocyanate (NCO) groups either at the termini or as pendant groups on the main chain. Accordingly, "NCO-terminated", as used herein, relates to a prepolymer that has free isocyanate groups at its terminus/termini.

"Diene-functionalized" and "dienophile-functionalized", as used herein, relate to polyurethanes that comprise diene and dienophile groups, respectively. Said groups may be terminal groups or may be pendant groups. In various embodiments, such polyurethanes are obtained by reacting a prepolymer having free isocyanate groups with an NCO-reactive compound comprising a diene and dienophile group, respectively. Thus, functionalized polyurethanes are obtained in which one, more than one or all free isocyanate groups are reacted and "converted" into dienes and dienophiles, respectively. Depending on the position of the reacted isocyanate group, the diene and dienophile groups may be terminal groups, forming a diene- or dienophile-terminated polyurethane, or pendant groups. In various alternative embodiments, the dienes and dienophiles, respectively, can be incorporated into the polyurethanes by different means, for example via the utilization of special diols or polyols and diene/dienophile-containing soft segments during prepolymer synthesis. Generally, polyols with diene or dienophile side chains may be prepared by reacting diene/dienophile precursors with a functionalized polyol.

"Diene", as used herein, relates to any compound comprising two carbon-carbon double bonds, in particular conjugated dienes, that is capable of undergoing a Diels-Alder reaction (4+2 cycloaddition) with a suitable dienophile. Exemplary dienes that can be used in accordance with the present invention are cyclopentadiene, maleimide and derivatives thereof, in particular maleimide and derivatives thereof.

"Dienophile", as used herein, relates to any compound comprising at least one carbon-carbon double bond that is capable of undergoing a Diels-Alder reaction (4+2 cycloaddition) with a suitable diene. Exemplary dienophiles that can be used in accordance with the present invention are substituted alkenes or heteroaryl compounds, including without limitation furan and derivatives thereof.

In various embodiments of the invention, steps (1)(a) and (3)(a) comprise forming an NCO-terminated polyurethane prepolymer from a reaction mixture comprising:
(a) at least one polyol, preferably with a number average molecular weight Mₙ in the range of 400 g/mol to 10000 g/mol measured by end group analysis (OH number) according to DIN 53240-2:2007-11; and
(b) at least one polyisocyanate, preferably at least one aliphatic di- and/or triisocyanate, wherein the at least one polyisocyanate is used in molar excess relative to the hydroxy groups of the at least one polyol of the reaction mixture to obtain an NCO-functional polyurethane prepolymer.

In various other embodiments of the inventive method, in steps (1)(b) or (3)(b) where a first or second polyisocyanate is reacted with a compound comprising at least two NCO-reactive groups and at least one diene and at least one dienophile groups, respectively, the polyisocyanate comprises at least one polyisocyanate, preferably at least one aliphatic di- and/or triisocyanate, wherein the at least one polyisocyanate is used in molar excess relative to the NCO-reactive groups of its reaction partner.

In such embodiments, the compound comprising at least two NCO-reactive groups and at least one diene and at least one dienophile groups, respectively, may be a polyol such as those defined herein which is further substituted with a diene, such as maleimide, or dienophile, such as furan, group.

In addition to the compound comprising at least two NCO-reactive groups and at least one diene or at least one dienophile group, at least one additional compound comprising two or more NCO-reactive groups but not including diene or dienophile groups, preferably at least one polyol, such as those described herein, may be used.

The at least one polyol is in various embodiments a non-functionalized polyol, e.g. contains no functional groups besides the hydroxyl groups or, optionally, the diene or dienophile group(s). It may be at least one polyester polyol, at least one polyether polyol, or at least one polycarbonate polyol, at least one polysiloxane polyol, at least one (hydrogenated) polybutadiene polyol or, preferably, a mixture thereof, in particular a mixture of at least two polyether polyols or at least one polyether polyol and at least one polyester polyol. It is understood that when in the following reference is made to specific polyols or polyol types, the invention is intended to encompass that polyols functionalized to include diene or dienophile groups, as described herein.

Polyester polyols that are useful in the processes described herein include those that are obtainable by reacting, in a polycondensation reaction, dicarboxylic acids with polyols. The dicarboxylic acids may be aliphatic, cycloaliphatic or aromatic and/or their derivatives such as anhydrides, esters or acid chlorides. Specific examples of these are succinic acid, glutaric acid, adipic acid, pimelic acid, suberic acid, azelaic acid or sebacic acid, phthalic acid, isophthalic acid, trimellitic acid, phthalic acid anhydride, tetrahydrophthalic acid anhydride, glutaric acid anhydride, maleic acid, maleic acid anhydride, fumaric acid, dimeric fatty acid and dimethyl terephthalate. Examples of suitable polyols are monoethylene glycol, 1,2-propanediol, 1,3-propanediol, 1,4-butanediol, 3-methylpentane-1,5-diol, neopentyl glycol (2,2-dimethyl-1,3-propanediol), 1,6-hexanediol, 1,8-otaneglycol cyclohexanedimethanol, 2-methylpropane-1,3-diol, dithyleneglycol, triethyleneglycol, tetraethyleneglycol, polyethyleneglycol, dipropyleneglycol, polypropyleneglycol, polypropyleneglycol, dibutyleneglycol and polybutyleneglycol. Alternatively, they may be obtained by ring-opening polymerization of cyclic esters, preferably ε-caprolactone. Preferred are crystalline/semicrystalline polyols, such as, for example, esters of 1,4-butanediol with adipic acid.

In various embodiments, the polyester polyol has a melting temperature Tₘ > 0°C, preferably > 40 °C and/or has a number average molecular weight Mₙ in the range of 400 to 5000, preferably 500 to 3000 g/mol, more preferably 800 to 2500 g/mol, most preferably 1000 to 2000 g/mol.

The polyether polyol may be a polyalkylene glycol homo- or copolymer, preferably a polypropylene glycol homo- or copolymer, a polyethylene glycol homo- or copolymer, a polytetramethylene glycol homo- or copolymer, or a polypropylenglycol/polyethyleneglycol block copolymer.

In various embodiments, the polyether polyol has a number average molecular weight of 1000 to 4000, preferably 1000 to 3000 g/mol.

Suitable polycarbonates can be obtained by reaction of carbon acid derivatives, e.g. diphenyl carbonate, dimethyl carbonate or phosgene with diols. Suitable examples of such diols include ethylene glycol, 1,2- and 1,3-propanediol, 1,3- and 1,4-butanediol, 1,6-hexanediol, 1,8-octanediol, neopentyl glycol, 1,4-bishydroxymethyl cyclohexane, 2-methyl-1,3-pro-panediol, 2,2,4-trimethyl pentanediol-1,3, dipropylene glycol, polypropylene glycols, dibutylene glycol, polybutylene glycols, bisphenol A, tetrabromobisphenol A as well as lactone-modified diols. The diol component preferably contains 40 to 100 wt. % hexanediol, preferably 1,6-hexanediol and/or hexanediol derivatives. More preferably the diol component includes examples that in addition to terminal OH groups display ether or ester groups.

The hydroxyl polycarbonates should be substantially linear. However, they can optionally be slightly branched by the incorporation of polyfunctional components, in particular low-molecular polyols. Suitable examples include glycerol, trimethylol propane, hexanetriol-1,2,6, butanetriol-1,2,4, trimethylol propane, pentaerythritol, quinitol, mannitol, and sorbitol, methyl glycoside, 1,3,4,6-dianhydrohexites.

Suitable polycarbonate polyols are, without limitation, those obtainable under the trademark names Desmophen® C3200 (Bayer) and Kuraray® C2050 (Poly-(3-methyl-1,5-pentanediol, 1,6-hexanediol)carbonate; Kuraray).

In various embodiments, the reaction mixture includes at least one amorphous polyol, preferably polyether polyol, such as polypropylene glycol or poly THF, with a number average molecular weight Mₙ in the range of 400 g/mol to 5000 g/mol, a crystallinity of less than 10 % and a glass transition temperature T_{g} in the range of -120°C to 40°C, preferably -70°C to 30°C, the crystallinity and the glass transition temperature as determined by differential scanning calorimetry (DSC) according to ISO 11357.

The amorphous polyols that can be used in accordance with the embodiments described herein are preferably polyether polyols and/or have a number average molecular weight Mₙ in the range of 400 g/mol to 5000 g/mol, preferably 500 to 3000 g/mol, more preferably 800-2500 g/mol, most preferably 1000 to 2000 g/mol. "Amorphous", as used herein in relation to the polyols, means that the polyol has a crystallinity of less than 10 %, preferably less than 5 %, more preferably less than 2 %, as determined by differential scanning calorimetry (DSC) according to ISO 11357. The amorphous (polyether) polyols furthermore have a glass transition temperature T_{g} in the range of -120°C to 40°C, preferably -70°C to 30°C, again as determined by differential scanning calorimetry (DSC) according to ISO 11357. Below the glass transition temperature, amorphous polymers are brittle and rigid. This property is due to the immobility of the "frozen" polymer chains. When the glass transition temperature is exceeded the molecular chains become movable relative to one another and the polymer softens, the degree of softening depending on the type of the polymer, the molecular weight of the polymer and the temperature. Amorphous polymers, as compared with (semi-)crystalline polymers, show only a glass stage in the DSC measurement according to ISO 11357 during the transition from the brittle, rigid state to the softened state. A melt peak indicating a semi-crystallinity of the polymer does not occur in the DSC measurements.

In various embodiments, the reaction mixture also includes one crystalline or semicrystalline polyol. The crystalline or semicrystalline polyol is preferably a polyester polyol or polycarbonate and may have a number average molecular weight Mₙ in the range of 400 g/mol to 5000 g/mol, preferably 500 to 3000 g/mol, more preferably 800-2500 g/mol, most preferably 1000 to 2000 g/mol. "Crystalline", as used herein in relation to the (polyester) polyols, relates to a crystallinity of at least 90%, preferably at least 95%, as determined by differential scanning calorimetry (DSC) according to ISO 11357. Similarly, "semicrystalline", as used herein in relation to the (polyester) polyols, means that they have a crystallinity of at least 50 %, preferably at least 70 %, but less than 90%. Semicrystalline (polyester) polyols thus comprise crystalline and non-crystalline, i.e. amorphous, regions. The crystalline and semicrystalline (polyester) polyols useful in the described mixtures may have a melting temperature Tₘ in the range of 40°C to 220°C, preferably >40°C to <160°C, more preferably >40°C to 80°C, most preferably >40°C to 60°C, again as determined by differential scanning calorimetry (DSC) according to ISO 11357 with a heating rate of 20K/min. The same method can be used for the determination of the melt enthalpy, wherein for the determination of the melt enthalpy the results of the second heating cycle are used. In various embodiments of the invention, the polyols have melt enthalpies of more than 90 J/g, preferably more than 115 J/g. If no standard reference of the polyol with known crystallinity for determination via DSC is available, known alternative methods, such as X-ray diffractometry, may be used.

In embodiments, where a combination of amorphous polyols, preferably polyether polyols, and crystalline/semicrystalline polyols, preferably polyester polyols or polycarbonates, is used, the molar ratio of the amorphous polyol to the crystalline/semicrystalline polyol may be in the range of 1:4 to 10:1, preferably 1:3 to 5:1, more preferably 1:2 to 2:1.

The reaction mixture may comprise further polyols, in particular diols. Such diols may be monomeric diols ortriols, such as 1,4-butanediol, 1,5-pentanediol, neopentylglycol, 1,6-hexanediol and mixtures of at least two of the afore-mentioned diols.

The reaction mixture may further comprise polyols that are hydroxy-functionalized polymers, for example hydroxy-functionalized siloxanes. Exemplary siloxanes that may be used are hydroxy-functionalized polydimethylsiloxanes, in particular in liquid form, such as those commercially available under the name Tegomer® H-Si 2311 (Evonik, Germany) having a molecular weight Mn of about 2200 g/mol. Suitable polydimethylsiloxane (PDMS) polyols are, for example, described in US 6794445 B2. They may be used in amounts of up to 60 wt.-% based on the total weight of the polyols used and typically have low T_{g} values, for example in the range of from -150 to -100 °C.

The reaction mixture may further comprise at least one stabilizer.

The term "stabilizer", as used herein, relates to a class of molecules that can stabilize the hydrophobic droplets in a water-based (oil-in-water, abbreviated as o/w) dispersion or emulsion, i.e. prevent coagulation or coalescence. The stabilizer molecules may bind to, adhere to or associate with the droplet surface. The peptides may act as additional the stabilizer molecules. The term "stabilizer", as used herein, furthermore relates to molecules that have a HLB value > 10, preferably > 12 and more preferably >15 according to Griffin and/or a water solubility of > 10 g/L, preferably > 50 g/L, more preferably > 100 g/L and most preferably > 150 g/L. Furthermore species which are able to carry an electric charge in water in a pH regime between pH3 (±1) to pH 11 (±1) are considered as stabilizers. Examples are molecules containing an anionic or latent anionic moiety, e.g. sulfate, sulfonate, phosphate, phosphonate or carboxylic groups without being limited to these. Additionally, molecules containing cationic or latent cationic groups, like quaternary or tertiary amine groups, without being limited to these, are also "stabilizers" in the sense of the present invention.

Generally, stabilizer molecules as known in the prior art comprise a hydrophilic and a hydrophobic part, with the hydrophobic part interacting with the droplet and the hydrophilic part be exposed to the solvent. Commonly used stabilizers are surfactants and may bear an electric charge, for example may be anionic surfactants or cationic surfactants, or may, alternatively, be non-ionic.

However, to avoid the use of external surfactants and also due to the increased stability provided, recently reactive stabilizer compounds that are built into the polyurethane polymer during (pre)polymer formation have become more popular, as these provide for (self-)emulsifiable polyurethanes which under agitation, e.g. high sheer emulsification, or spontaneously form stable dispersions in water without the assistance of external emulsifiers.

These reactive stabilizers comprise isocyanate-reactive functionalities in order to become incorporated in the polyurethane structure. Preferred species are polyols, most preferably diols, or a mixture of different polyols and/or diols.

Known non-ionic polymeric stabilizers have an average molecular weight Mₙ of about 4000 g/mol, preferably up to about 3000 g/mol, more preferably up to about 1000 g/mol. Suitable non-ionic ethylene glycol/propylene glycol stabilizers are for example those commercially available under the trademark name Pluronic® from BASF, for example Pluronic PE3500. The non-ionic stabilizers may, in various embodiments, have HLB (hydrophile lipophile balance) values between 6 and 20, preferably > 10. The HLB values are calculated by calculating the hydrophilic portion of the molecule by dividing the molecular weight of the hydrophilic part of the molecule, such as the alkylene oxide, by the total molecular weight of the molecule and then dividing the obtained percentage by 5.

While the afore-mentioned stabilizers are non-ionic, alternative stabilizers may be modified by anionic or cationic groups. The presence of such charged groups may increase the stability of the dispersed polymer droplets or particles. Suitable anionic groups include, but are not limited to acidic groups, such as carboxylic acid or sulfonic acid groups and their respective salts. Concrete compounds suitable as anionic stabilizers in the sense of the present invention are 2,2-bis(hydroxymethyl)propionic acid (dimethylol propionic acid; DMPA) and sulfonated polydiols with a molecular weight M_{w} in the range of up to 1000 g/mol, preferably up to 500 g/mol. Such sulfonated polydiols, for example propoxylated 1-methyl-2-methylol-3-hydroxy-1-propanesulfonate with a molecular weight M_{w} of about 430 g/mol, are commercially available under the name GS-7Q (Yedang G & Co. Ltd).

Suitable cationic groups include, but are not limited to ammonium groups, in particular quaternary ammonium groups. Particularly preferred are alkoxylated, preferably ethoxylated or propoxylated, dialkylammonium diols, such as ethoxylated cocoalkylmethyl ammonium methanesulfonate. Such compounds are commercially available under the trademark name Rewoquat® CPEM from Evonik.

The term "reaction mixture", as used herein, relates to the mixture of the polyols, optionally including the compounds including NCO-reactive groups and diene or dienophile groups, and the polyisocyanate(s). "Polyol mixture", as used herein in relation to the mixture comprising the polyols, relates to a mixture comprising the at least one polyol and, optionally, any additional polyols, including polyhydroxy-modified polymers of various types or the diene- or dienophile-containing NCO-reactive compounds (preferably diene- or dienophile-containing polyols), that may be present.

It is preferred that the polyol mixture does not contain any organic solvents or surfactants and no further additives, i.e. consists only of the polyols, preferably those defined above.

In various embodiments, the polyol mixture comprises about 20 to about 100 wt.-%, preferably 30 to 100 wt.-%, of the polyol(s), preferably a mixture of different polyols, for example of polyester polyols and polyether polyols, relative to the weight of the polyol mixture. If present, the stabilizer is usually contained in amounts of about 1 to 30 wt.-%, preferably 10 to 20 wt.-%, relative to the weight of the polyol mixture.

The other reactant employed in the formation of the polyurethane (pre)polymer is a polyisocyanate. Any compound which includes at least two isocyanate groups is within the contemplation of the present invention. It is preferable, however, that the polyisocyanate be a diisocyanate. The incorporation of small amounts of isocyanate with a functionality higher than two, in particular a triisocyanate, is also contemplated and may under certain circumstances even be advantageous. Such polyisocyanates can act as cross-linkers. In this case where the polyisocyanate acts as a crosslinker, polyisocyanates based on hexamethylene diisocyanate are preferred. Suitable diisocyanates include, without limitation, methylenediphenyl diisocyanate (MDI), toluene-2,4-diisocyanate (TDI), hexamethylene diisocyanate (HDI), polymeric diphenylmethane diisocyanate (PMDI), isophorone diisocyanate (IPDI), methylene-4,4-bis(cyclohexyl)diisocyanate (H12MD1) and mixtures thereof. Although both aliphatic and aromatic polyisocyanates are within the contemplation of the present invention, it is preferred that the polyisocyanate be an aliphatic polyisocyanate. Thus, in a particularly preferred embodiment, the polyisocyanate is an aliphatic diisocyanate. Among particularly preferred aliphatic diisocyanates are isophorone diisocyanate, hexamethylene diisocyanate, and mixtures thereof. Suitable polyisocyanates are, for example, commercially available under the trademark name Desmodur® from Bayer AG (DE).

The polyisocyanate is used in molar excess relative to the OH groups of all polyols present in the reaction mixture, the OH/NCO equivalent ratio preferably being 1:1.1 to 1:4, more preferably 1:1.2 to 1:1.3. The amount of the at least one polyisocyanate in the reaction mixture is typically in the range of 10 to 20 wt.-% relative to the reaction mixture. The remainder of the reaction mixture may be made up by the polyol mixture, as defined above.

Providing the polyol mixture may include the step of mixing the polyols and heating the mixture. The heating may be required in case the polyols employed are solid at room temperature and need to be melted to form the polyol mixture. In preferred embodiments, the polyols are combined and heated to about 70 to 95°C, for example about 75°C, while stirring the mixture under vacuum to dry. After the mixing, the mixture may be cooled to 60°C for the addition of the isocyanates.

The polyol mixture is subsequently combined with at least one polyisocyanate in the reaction mixture to form the (pre)polymer. The (pre)polymer reaction usually occurs at elevated temperature, preferably in the range of between about 70 °C and about 95 °C, more preferably about 80 °C, over a period of between about 1 and about 24 hours. The reaction is typically carried out in the presence of a catalyst that is added, preferably a tin-, bismuth- or zinc-based catalyst, more preferably dimethyldineodecanoatetin, for example commercially available under the trade name Fomrez UL28 (Momentive Performance Materials GmbH, Germany). Alternative catalysts of high reactivity are Bismuth-neodecanoate and Zn-neodecanoate available under the trade names BorchiKat 315 and BorchiKat 0761 (OMG Borchers GmbH, Germany). In preferred embodiments of the invention, the reaction mixture thus further comprises a catalyst as defined above

The reaction continues until the free isocyanate content reaches or comes very close to the calculated value, as determined by standard titration with dibutylamine. Preferred values for the free isocyanate content in the (pre)polymer are in the range between 0.2 and 3 wt.-%, preferably 1 to 2 wt.-% relative to the total amount of polyols, including the stabilizer(s), and polyisocyanate in the mixture.

"About", as used herein, relates to ± 10 %, preferably ± 5 % of the numerical value to which it refers. "About 70 °C" thus relates to 70 ± 7, preferably 70 ± 3.5 °C.

As described above, in the polyurethane (pre)polymer-forming reaction the polyisocyanate is reacted in a concentration in excess of the stoichiometric concentration required to completely react with the hydroxyl groups. The excess used may include an OH/NCO equivalent ratio of 1:1.1 to 1:4. Preferably, the amount of the polyisocyanate is 20 % to 150 % in excess of the stoichiometric concentration required to completely react with the hydroxyl groups.

In various embodiments, steps (1)(a) and (3)(a) are carried out by adding the compound comprising at least one, preferably only one, NCO-reactive group, such as a hydroxyl group, and at least one diene and dienophile group, respectively, to the formed prepolymer once the desired NCO content has been reached. The addition is preferably done without further purification of the prepolymer. The reaction temperature can be maintained in the range given above for the prepolymer forming reaction.

The compound comprising a hydroxyl and at least one diene group preferably comprises at least one maleimide group, and is more preferably an N-hydroxyalkyl maleimide, such as, for example and without limitation, N-(2-hydroxyethyl)maleimide. In some embodiments, in the diene/maleimide capping reaction, the prepolymer is reacted with a concentration of the diene/maleimide compound that is in excess of the stoichiometric concentration required to completely react with the isocyanate (NCO) groups of the prepolymer. The excess may be a molar excess and may include an NCO/(diene/maleimide) equivalent ratio of 1:1.1 to 1:4, preferably about 1:2.5. In some embodiments, the amount of the diene/maleimide groups is 20 % to 150 % in excess of the stoichiometric concentration required to completely react with the NCO groups. In such embodiments, a fully diene/maleimide-functionalized (in case of an NCO-terminated prepolymer a fully diene/maleimide-endcapped) polyurethane polymer can be obtained. In various other embodiments, the concentration of the diene/maleimide compound is lower than the stoichiometric concentration required to completely react with the NCO groups of the prepolymer. In the latter embodiments, a (partial) chain extension as described above can be carried out. Suitable chain extension reagents are those having two or more NCO-reactive groups, as described in more detail below.

Similarly, the compound comprising a hydroxyl and at least one dienophile group preferably comprises at least one furan group, and is more preferably an 2-hydroxyalkyl furan, such as, for example and without limitation, 2-hydroxymethyl-furan. In some embodiments, in the dienophile/furan capping reaction, the prepolymer is reacted with a concentration of the dienophile/furan compound that is in excess of the stoichiometric concentration required to completely react with the isocyanate (NCO) groups of the prepolymer. The excess may be a molar excess and may include an NCO/(dienophile/furan) equivalent ratio of 1:1.1 to 1:4, preferably about 1:2.5. Preferably, the amount of the dienophile/furan groups is 20 % to 150 % in excess of the stoichiometric concentration required to completely react with the NCO groups. In such embodiments, a fully dienophile/furan-functionalized (in case of an NCO-terminated prepolymer a fully dienophile/furan-endcapped) polyurethane polymer can be obtained. In various other embodiments, the concentration of the dienophile/furan compound is lower than the stoichiometric concentration required to completely react with the NCO groups of the prepolymer.

The successful functionalization of the prepolymer by maleimide groups or furan groups can be monitored by Infrared spectroscopy (IR).

Subsequently, the formed diene- or dienophile-functionalized polymer may be dissolved in a suitable organic solvent, including, without limitation, ethyl acetate or acetone. The organic solvent is preferably water-immiscible.

In various embodiments, the equivalent weight of the introduced functional diene or dienophile group in the polymer ranges from 500 to 50000 g/mol, preferably 500 to 30000 g/mol, more preferably 1000 to 10000 g/mol. The functionalized polymer may have a number average molecular weight Mₙ of 2500 or more g/mol, preferably 3000 or more g/mol, more preferably 5000 or more g/mol. The upper limit of the average molecular weight of the polymer may, in some embodiments, range between 30000-50000.

In various embodiments and if necessary, the functionalized polymer may be neutralized at this stage by using a suitable neutralization agent. Typically, this is only necessary if an ionic stabilizer is used. For example, in case an anionic acidic stabilizer is used, an amine base, such as triethylamine may be used for neutralization.

The thus formed diene- or dienophile-functionalized polymer is then dispersed in a continuous aqueous phase in steps (2) and (4), respectively. The "continuous aqueous phase," as used herein, relates to a dispersion medium which preferably contains as a solvent predominantly (>50 vol.-%, preferably >80 vol.-%, more preferably >90 vol.-%) or exclusively (i.e. 100 vol.-%) water, i.e. contains no other liquid solvent. The aqueous phase may however further comprise solutes, for example salts and buffer substances, but is preferably water essentially without any other solvents or solutes.

The dispersion may be achieved under vigorous agitation. In various embodiments, the polymer is used in form of a solution of the polymer in an organic solvent, with the solution then being emulsified in water. The dispersing step may be carried out at elevated temperature, for example in the range of about 30 to 60 °C, for example at about 40 °C. The dispersing step may be done by mechanical stirring alone or by dispersing devices, such as high-pressure homogenizers, microfluidizers or rotor-stator dispersing machines, such as an ultra-turrax device.

The dispersing step may include emulsifying the polyurethane polymer in liquid form into a continuous aqueous phase, preferably water, to form an emulsion

For the dispersing step, the polymer is preferably used in liquid form, i.e. it is either liquid at room temperature or, if it is solid at room temperature, it is melted or dissolved in a suitable organic solvent, such as, without limitation, acetone or ethyl acetate. If an organic solvent is used, this may be removed later from the dispersion by known means, for example by evaporation using a rotary evaporator. In such solutions of the polymer, the polymer and organic solvent are preferably used in weight ratios of 2:1 to 1:4, preferably about 1:1.

The term "emulsion", as used herein, relates to oil-in-water (O/W) emulsions, i.e. emulsions in which water is used in excess and is the continuous medium. In the described processes, stable droplets/particles of the PU polymer with a diene/dienophile functionalization are obtained, which have typically a size between 50 and 1000 nm, preferably between 50 and 500 nm, more preferably between 100 and 400 nm. The particles preferably have 10² to 10¹, more preferably 10³ - 10⁶ diene/dienophile groups per particle.

The processes of the invention may further comprise a step of separating the functionalized polyurethane polymer particles from any unreacted compounds. This may for example be achieved by dialysis or any other suitable technique, such as chromatography.

Before step (5) or simultaneously with step (5), the diene/dienophile functionalized polymer, in particular those polymers that are not completely functionalized, may be further reacted with a suitable chain extension agent or water to react all free NCO groups. To achieve this, a suitable chain extension agent is added to the emulsion of the polymer. Suitable chain extension agents are compounds that comprise at least two NCO-reactive groups. "NCO-reactive groups", as used herein, relates to functional groups that are reactive towards isocyanate, i.e. NCO groups, such as hydroxyl groups, amino groups, thiol groups and carboxyl groups. Exemplary chain extension agents include, without limitation, diamines, such as hydrazine, alkylene diamines or cycloalkylene diamines, preferably ethylene diamine, isophorone diamine, piperazine, polyether amine, and diols, such as butane diol or 2-butyl-2-ethyl-1,3-propane diol. Furthermore, small amounts of tri- or higher functional amines can be used to introduce branching and crosslinking and thus increase the heat resistance of the materials. The chain extension can be carried out until total conversion of free isocyanate groups, i.e. until no free NCO groups are detectable. For the chain extension reaction, the presence of a catalyst and/or elevated temperatures may be necessary.

In step (5), the diene-functionalized polyurethane dispersion and the dienophile-functionalized polyurethane dispersion are combined. The combined dispersions form a latent reactive polyurethane dispersion, as the reactive diene and dienophile groups are separated by water molecules as long as the polymer particles are stably emulsified, which is stable over extended periods of time.

The aqueous polyurethane dispersion formed preferably has a solid content of 5 to 68 wt.-%, preferably 10 to 60 wt.-%, more preferably 10 to 55 wt.-% and most preferably 10 to 50 wt.-%.

During use of the dispersion, typically involving application and drying, the previously separated polymer particles come into contact with each other and after the interdiffusion of polymer chains a reaction between the diene and dienophile groups in a Diels-Alder reaction occurs, thus cross-linking the polymer chains and providing a cross-linked polymer (film). Interestingly, the reaction is reversible (retro-Diels-Alder reaction) at elevated temperature, thus rendering the resulting polymer films thermoresponsive. This property can be used for bonding and debonding of substrates or the self-healing of polymer films.

As already mentioned above, the functionalized polyurethane particle dispersions obtainable by the described processes form also part of the present invention.

The aqueous functionalized polyurethane dispersions may be used in various fields, such as, without limitation, coating and adhesive products and compositions, and adhesive applications. Further applications are laundry detergents, textile care/treatment agents and cosmetic compositions. Such compositions can contain further ingredients all of which are well-known in the field. Accordingly, such uses as well as the compositions as such are also encompassed by the present invention.

The functionalized PUDs of the invention can advantageously be used in coatings, paints and varnishes, adhesives or bonding agents. The present invention therefore also encompasses coatings, varnishes and paints, adhesives or glues as well as (laundry) detergents, textile treatment agents and cosmetic compositions that contain the functionalized polyurethane dispersions of the invention.

Also contemplated are methods for forming a polymer film, comprising applying the compositions containing the PUDs of the invention onto a carrier and drying the composition to form a polymer film. The invention also encompasses the thus obtained polymer films.

The invention further includes methods for bonding two substrates to each other, comprising:
(a) providing two substrates,
(b) disposing an adhesive composition comprising the dispersions described herein on one or both substrate(s),
(c) contacting the substrates so that the adhesive composition is disposed between the substrates, forming an assembly,
(d) curing the adhesive by drying.

The thus obtained bonded substrates or assemblies also form part of the invention.

It is understood that all embodiments disclosed herein in relation to the processes and methods are similarly applicable to the disclosed dispersions, compositions, and uses, and vice versa.

The following examples are given to illustrate the present invention. Because these examples are given for illustrative purposes only, the invention should not be deemed limited thereto.

### Examples

### Materials and Methods

### Chemicals

Polypropylene glycol (Voranol 2000 L, abbreviated here PPO, M = 2000 g/mol) was obtained from Dow Chemical Canada, Inc., Canada. Poly(oxypropylene,oxyethylene) glycol (K HN 8200, abbreviated here PEOE, M = 1941 g/mol) was obtained from Hannong Chemicals. The sulfonated Diol (GS-7Q, abbreviated here SOH, M = 425 g/mol) was obtained from Yedang G&B Co., Ltd., Chungbuk, Korea. Isophorone diisocyanate (IPDI, M = 222.29 g/mol) was obtained from Merck, Germany. The catalyst (Fomrez UL-28) was obtained from Momentive Performance Materials GmbH, Germany, as a 50 wt.-% solution in acetone. N-(2-hydroxyethyl)maleimide (HEMI, M = 141.12 g/mol) was obtained from Shanghai Jinshan Pharmaceutical. Furfuryl alcohol (FUA) was obtained from Fluka. The solvents toluene, chloroform, and acetone were obtained from Sigma-Aldrich Chemie GmbH, Germany. Acetone was dried over molecular sieve. Unless otherwise specified, chemicals were used as received.

### DLS

Dynamic Light Scattering (DLS) was used to determine z-average particle sizes according to ISO 22412:2008. For Dynamic light scattering, a NICOMP 380 Particle Sizer from Particle Sizing Systems was used (635 nm Laser; scattering angle 90°). Samples were diluted prior measurement.

### GPC

GPC was conducted in THF on Styragel columns HR1, HR3 and HR5 from Waters at 35 °C.

### Rheology

Rheology measurements were performed on an Anton Paar MCR 302 Rheometer with parallel plate geometry (25 mm diameter).

### Example 1

### Synthesis of furan-functionalized PU prepolymer

PPO (157.74 g), PEOE (8.00 g) and SOH (8.00 g) were heated to 70-80 °C until molten, then vacuum (pressure < 0.1 mbar) was applied for 30-90 minutes until a constant pressure of 1 mbarwas reached to remove traces of water. N₂ was fed into the flask and IPDI (26.26 g) and 30 µl of catalyst (Fomrez UL-28, 50 wt.-% in acetone) were added. After the initial increase in temperature, the reaction was carried out at 80-85 °C. The NCO content was checked every half hour using the back titration method until it had reached the theoretical residual value of 0.7%. Then, FUA (2.81 g) was added in excess to react with the remaining NCO groups. The obtained polymer had a molecular weight M_{w} of 25100 g/mol as determined by GPC.

### Synthesis of maleimide-functionalized PU prepolymer

PPO (157.74 g), PEOE (8.00 g) and SOH (8.00 g) were heated to 70-80 °C until molten, then vacuum (pressure < 0.1 mbar) was applied for 30-90 minutes until a constant pressure of 1 mbar was reached to remove traces of water. N₂ was fed into the flask and IPDI (26.26 g) and 30 µl of catalyst (Fomrez UL-28, 50 wt.-% in acetone) were added. After the initial increase in temperature, the reaction was carried out at 80-85 °C. The NCO content was checked every half hour using the back titration method until it had reached the theoretical residual value of 0.7%. Then, HEMI (4.31 g) was added in excess to react with the remaining NCO groups. The obtained polymer had a molecular weight M_{w} of 28300 g/mol as determined by GPC.

### Dispersing

Each of the obtained functionalized polymers was dissolved in dried acetone (dried over a molecular sieve of 3A) in a mass ratio of 1:1. A dispersion was formed by adding the solution to water using an UltraTurrax T25 (IKA) operating at 11000 rpm for 3 min. Then, the dispersion was passed through a Microfluidizer M-100Y (Microfluidics, 11000 psi, 4 passes, chamber configuration H210Z and H230Z) at 40°C.

The solvent acetone was then evaporated in a rotary evaporator at elevated temperature and reduced pressure.

Maleimide- and furan-functionalized particles were obtained with an average particle size of 245 nm and standard deviations of 12 and 15%. Solid contents of the dispersions were 16.4 % solids in the furan-functionalized PU dispersion and 15.6 % solids in the maleimide-functionalized PU dispersion.

### Combination

The two dispersions were combined in a molar ratio of 1:1 to yield a dispersion with a solid content of 16.0 % and stored at 50°C. Over a period of 28 days the particle size, as determined by DLS, did not change, as shown in Table 1 below. This shows that the obtained dispersions are stable.

**Table 1**

| Time (d) | dᵥ / nm | S.D. |
|---|---|---|
| 0 | 250 | 0.17 |
| 7 | 249 | 0.17 |
| 28 | 249 | 0.19 |

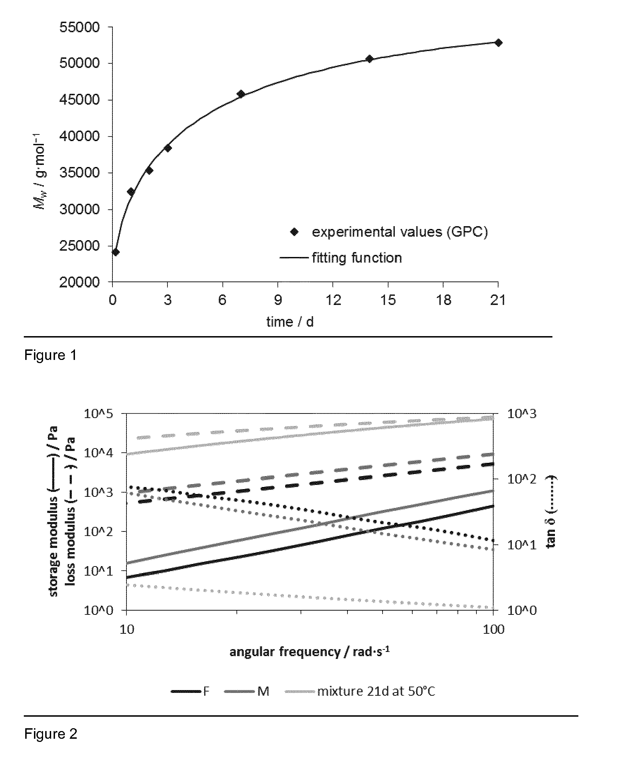

The mixture stored for 21 days at 50°C was then used for film formation by applying it to a substrate and drying. The obtained GPC values (M_{w}) are shown in **Figure 1**. The results show a clear increase in molecular weight over time, indicating the occurrence of a cross-linking reaction between the maleimide and furan groups.

Rheological measurements of the mixture of the two dispersions after 21 days at 50°C showed increases in storage and loss moduli and a decrease of tanδ caused by the reaction (**Figure 2**).

### Reverse reaction

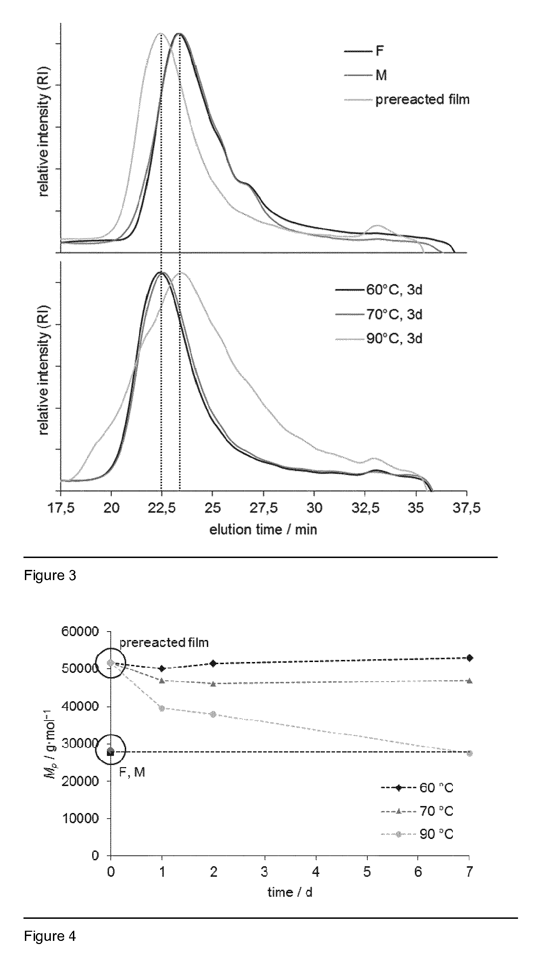

A pre-formed polymer film dried and stored at 50°C for 7 days was incubated for 3 days at 60, 70 and 90°C, respectively. **Figure 3** shows GPC elution profiles for the PU films before and after the Diels-Alder reaction as well as the elution profiles of the educts (single dispersions). The results demonstrate that at 90°C a retro-Diels-Alder reaction occurred.

**Figure 4** shows the development of the peak molecular weight at different temperatures over time and confirms that at 90°C a reverse reaction to the educts occurred.

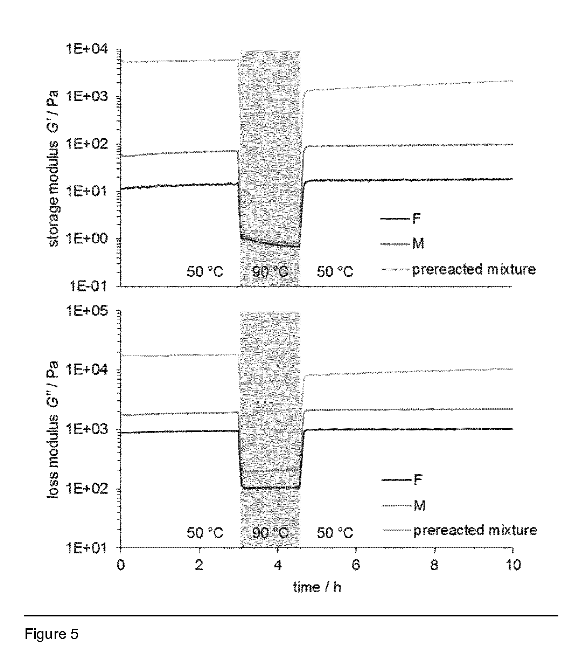

**Figure 5** shows the rheological properties (storage and loss modulus) of a pre-reacted mixture (1 week at 50°C) and the single dispersions at 50 and 90°C.

These results show that the materials are suitable for the production of self-healing polymer films and for bond/debond-on-command adhesives.

## Claims

1. Process for manufacturing an aqueous polyurethane dispersion (PUD) comprising at least one diene-functionalized polyurethane and at least one dienophile-functionalized polyurethane, the process comprising
(1)
(a) providing a first NCO-functional polyurethane prepolymer and reacting said first NCO-functional polyurethane prepolymer with a compound comprising at least one NCO-reactive group and at least one diene group, preferably a compound comprising one hydroxyl and at least one maleimide group, to obtain a diene-functionalized polyurethane; or
(b) reacting a first polyisocyanate with a compound comprising at least two NCO-reactive groups and at least one diene group, preferably a compound comprising at least two hydroxyl and at least one diene group, preferably maleimide group, to obtain a diene-functionalized polyurethane;
(2) dispersing said diene-functionalized polyurethane into a continuous aqueous phase to form a diene-functionalized polyurethane dispersion;
(3)
(a) providing a second NCO-functional polyurethane prepolymer and reacting said second NCO-functional polyurethane prepolymer with a compound comprising at least one NCO-reactive group and at least one dienophile group, preferably a compound comprising one hydroxyl and at least one furan group, to obtain a dienophile-functionalized polyurethane; or
(b) reacting a second polyisocyanate with a compound comprising at least two NCO-reactive groups and at least one dienophile group, preferably a compound comprising at least two hydroxyl and at least one dienophile group, preferably furan group, to obtain a dienophile-functionalized polyurethane;
(4) dispersing said dienophile-functionalized polyurethane into a continuous aqueous phase to form a dienophile-functionalized polyurethane dispersion; and
(5) combining the diene-functionalized polyurethane dispersion and the dienophile-functionalized polyurethane dispersion, thereby forming the aqueous polyurethane polymer dispersion.

2. The process according to claim 1, wherein steps (1)(a) and/or (3)(a) comprise forming an NCO-terminated polyurethane prepolymer from a reaction mixture comprising:
(a) at least one polyol, preferably with a number average molecular weight Mₙ in the range of 400 g/mol to 10000 g/mol; and
(b) at least one polyisocyanate, preferably at least one aliphatic di- and/or triisocyanate, wherein the at least one polyisocyanate is used in molar excess relative to the hydroxy groups of the at least one polyol of the reaction mixture to obtain an NCO-terminated polyurethane prepolymer.

3. The process according to claim 2, wherein
(1) the at least one polyisocyanate is used in molar excess relative to the hydroxy groups of the combined polyols, the OH/NCO equivalent ratio preferably being 1:1.1 to 1:4, and/or
(2) the at least one polyisocyanate is at least one diisocyanate or triisocyanate, preferably selected from the group consisting of isophorone diisocyanate (IPDI), hexamethylene diisocyanate (HDI), polymeric polyisocyanates based on IPDI or HDI, and mixtures thereof.

4. The process according to any one of claims 1 to 3, wherein the compound comprising at least one NCO-reactive group and at least one diene group is an N-hydroxylalkyl maleimide, preferably N-(2-hydroxyethyl)maleimide (HEMI).

5. The process according to any one of claims 1 to 4, wherein the compound comprising at least one NCO-reactive group and at least one dienophile group is a substituted alkene or heteroaryl, preferably a 2-hydroxylalkyl furan, more preferably 2-hydroxymethyl furan.

6. The process according to claim 1, wherein in step (1)(b) and/or (3)(b) the polyisocyanate comprises at least one aliphatic di- and/or triisocyanate, wherein the polyisocyanate is used in molar excess relative to the NCO-reactive groups of its reaction partner.

7. The process according to claim 1 or 6, wherein the compound comprising at least two NCO-reactive groups and at least one diene or at least one dienophile groups is a polyol substituted with a diene, preferably a maleimide, or dienophile, preferably furan, group.

8. The process according to any one of claims 1, 6 and 7, wherein in step (1)(b) and/or (3)(b) at least one further compound comprising two or more NCO-reactive groups but no diene or dienophile groups, preferably at least one polyol, is used.

9. The process according to any one of claims 1 to 8, wherein the dispersing steps (2) and (4) comprise emulsifying the functionalized polyurethane polymer into the continuous aqueous phase to form an emulsion.

10. Aqueous polyurethane dispersion obtainable according to a process of any one of claims 1 to 9.

11. Composition, preferably adhesive composition, coating composition, laundry detergent, textile care agent, or cosmetic composition, comprising the aqueous polyurethane dispersion according to claim 10.

12. Use of the aqueous functionalized polyurethane dispersion according to claim 10 in an adhesive composition, coating composition, laundry detergent, textile care agent, or cosmetic composition.
